# EUROPÄISCHE PATENTSCHRIFT

(11) **EP 0 765 154 B1**
(45) Veröffentlichungstag und Bekanntmachung des Hinweises auf die Patenterteilung: **20.01.1999**
(21) Anmeldenummer: 95923249.7
(22) Anmeldetag: 07.06.1995
(51) Int. Cl.: A61K 7/13

(54) **DIREKTZIEHENDES HAARFÄRBEMITTEL**
DIRECT-ACTING HAIR COLOURING AGENT
AGENTS DE COLORATION DIRECTE POUR CHEVEUX

(30) Priorität: 16.06.1994 DE 4421031
(43) Veröffentlichungstag der Anmeldung: 02.04.1997
(73) Patentinhaber: Henkel Kommanditgesellschaft auf Aktien, 40191 Düsseldorf (DE)
(72) Erfinder: HOLLENBERG, Detlef, D-40699 Erkrath (DE); MATZIK, Iduna, D-40699 Erkrath (DE)
(86) Internationale Anmeldenummer: EP9502192
(87) Internationale Veröffentlichungsnummer: WO9534273

(56) Entgegenhaltungen:
- EP-A- 0 470 381
- EP-A- 0 531 943
- EP-A- 0 547 790
- FR-A- 2 436 213
- GB-A- 2 259 717

## Beschreibung

Gegenstand der vorliegenden Erfindung sind Haarfärbemittel, die direktziehende Farbstoffe, kationische Polymere, bestimmte α-Hydroxysäuren und Ethercarbonsäure enthalten und die herkömmlichen direktziehenden Haarfärbemitteln im Hinblick auf wichtige färbetechnische Parameter überlegen sind.

Direktziehende Haarfarbstoffe erfreuen sich zunehmender Beliebtheit, da sie menschliche Haare im Gegensatz zu Oxidationshaarfärbemitteln auf schonende Art und Weise färben. Hinsichtlich färbetechnisch relevanter Parameter wie z.B. Farbintensität, Brillanz (Leuchtkraft) und Egalisierungsvermögen sind sie i.a. jedoch schlechter zu bewerten als Oxidationshaarfärbemittel.

Es besteht daher das Bedürfnis, direktziehende Haarfärbemittel hinsichtlich der o.g. Parameter zu verbessern. In der deutschen Patentschrift DE 29 36 934 werden Haarbehandlungsmittel offenbart, die u.a. ein kationisches Polymer und ein Tensid vom Typ Ethercarboxylat enthalten. Die dort beschriebenen Haarbehandlungsmittel verleihen dem Haar Weichheit, Geschmeidigkeit, leichte Entwirrbarkeit und Kämmbarkeit. Fakultativ können auch direktziehende Farbstoffe enthalten sein.

Es wurde nun gefunden, daß direktziehende Haarfärbemittel mit färbetechnisch hervorragenden Eigenschaften (Farbintensität, Brillanz und Egalisierungsvermögen) erhalten werden, wenn neben direktziehenden Farbstoffen kationische Polymere, bestimmte α-Hydroxysäuren und Ethercarbonsäuren enthalten sind.

Gegenstand der Erfindung ist daher ein wasserhaltiges Haarfärbemittel enthaltend
- einen direktziehenden Farbstoff in einer Menge von 0,01 bis 5,0 Gew.-%,
- ein kationisches Polymer in einer Menge von 0,05 bis 2,0 Gew. -%,
- eine α-Hydroxysäure ausgewählt aus der Gruppe Milchsäure. Zitronensäure und Weinsäure bzw. deren Alkali- oder Ammoniumsalzen in einer Menge von 0,05 bis 2,0 Gew.-%,
- eine Ethercarbonsäure der Formel I

   R¹ - O - (CH₂CH₂O)ₙ - CH₂COOH (1),
in der R¹ für eine Alkylgruppe mit 8 bis 22 C-Atomen und n für eine Zahl von 1 bis 15 steht, bzw. deren Alkali- oder Ammoniumsalze in einer Menge von 0,5 bis 8,0 Gew.-%, alle Gew.%-Angaben bezogen auf das gesamte Haarfärbemittel.

Sowohl kationische Polymere, α-Hydroxycarbonsäuren als auch Ethercarbonsäuren sind prinzipiell als Bestandteile von Haarfärbemitteln bekannt.

So offenbart die EP-A2-0 470 381 Haarfärbemittel mit direktziehenden Farbstoffen und kationischen Polymeren. Als fakultative Bestandteile werden weiterhin Substanzen zur Einstellung des pH-Wertes, z. B. α-Hydroxycarbonsäuren, und anionische Tenside, u. a. Ethercarbonsäuren, genannt. EP-A1-0 547 790 offenbart Farbemittel auf Basis direktziehender Farbstoffe und spezieller Siloxane. Als fakultative Komponenten werden weiterhin in der Beschreibung und den Beispielen kationischen Polymere, . α-Hydroxycarbonsäuren und Aniontenside, wie Ethercarbonsäuren, genannt. EP-A1-0 531 943, die Haarfärbemittel auf Basis von direktziehenden Farbstoffen sowie einer speziellen Tensidmischung betrifft, entspricht in ihrem für die vorliegende Erfindung relevanten Informationsgehalt der vorher genannten Schrift. Schließlich betrifft die GB-A-2259717 Haarfärbemittel mit sauren direktziehenden Farbstoffen und organischen Lösungsmitteln. Zur Einstellung des pH-Wertes können α-Hydroxycarbonsäuren, als weitere fakultative Komponenten Aniontenside, wie Ethercarbonsäuren, und kationische Polymere verwendet werden.

Keiner der genannten Druckschriften ist aber irgendein Hinweis zu entnehmen, daß eine Kombination von Vertretern dieser drei Substanzklassen die Färbeergebnissen von Haarfärbemitteln mit direktziehenden Farbstoffen insbesondere hinsichtlich Farbintensität und Farbbrillanz signifikant verbessern kann.

Besonders bevorzugt sind Haarfärbemittel, die als α-Hydroxycarbonsäure Weinsäure bzw. deren Alkali- oder Ammoniumsalze enthalten.

Als direktziehende Farbstoffe können alle üblicherweise in der Haarfärberei eingesetzten Direktfarbstoffe verwendet werden. Es sind dies z.B. Indoaniline, Indophenole, Nitrobenzolderivate, Anthrachinon-Farbstoffe, Triphenylmethanfarbstoffe und Azofarbstoffe. Vertreter dieser Farbstoffklassen sind beispielsweise die unter den Bezeichnungen HC Yellow 2, HC Yellow 4, Hydroxyethyl-2-nitro-p-toluidin, HC Yellow No. 6, Basic Yellow 57, Disperse Orange 3, HC Red 3, HC Red BN, HC Red No. 13, HC Red No. 10, HC Red No. 11, 2-Chlor5-nitro-N-hydroxyethyl-p-phenylendiamin, Basic Red 76, HC Blue 2, HC Blue No. 12, Disperse Blue 3, Basic Blue 99, HC Violet 1, Disperse Violet 1, Disperse Violet 4, Disperse Black 9, Basic Brown 16, Pikraminsäure und Rodol 9 R bekannten Verbindungen. Weitere geeignete direktziehende Farbstoffe sind beispielsweise die Verbindungen 3-Nitro-4-ethylaminobenzoesäure, 1,2,3,4-Tetrahydro-6-nitro-chinoxalin, 2-Nitro-4-amino-4'-dimethylaminodiphenylamin-2'-carbonsäure und 2-Nitro-4-aminodiphenylamin-2'-carbonsäure.

Als kationische Polymere kommen praktisch alle wasserlöslichen Polymeren (d.h. wenigstens 0,1 Gew.-% des Polymeren in Salzform sind bei 20° C in Wasser löslich), bevorzugt im Molekulargewichtsbereich von 1000 bis 3 000 000, in Frage, die entweder in der Polymerkette freie oder alkylsubstituierte Aminogruppen oder quartäre Ammoniumgruppen enthalten oder an die Polymerkette direkt oder über Zwischenglieder gebundene, sekundäre oder tertiäre Aminogruppen oder quartäre Ammoniumgruppen tragen. Diese Aminogruppen oder quartären Ammoniumgruppen können auch Glieder von 5- oder 6-gliedrigen Ringsystemen sein, z.B. von Morpholin, Piperidin-, Piperazin- oder Indazol-Ringen. Zahlreiche Beispiele für solche wasserlöslichen kationischen Polymeren sind z.B. in US-PS 4.240.450 näher beschrieben. Darüber hinaus sind zahlreiche weitere wasserlösliche kationische Polymeren literaturbekannt. Bevorzugt geeignet sind wasserlösliche Homo- oder Mischpolymerisate mit Einheiten der allgemeinen Formel 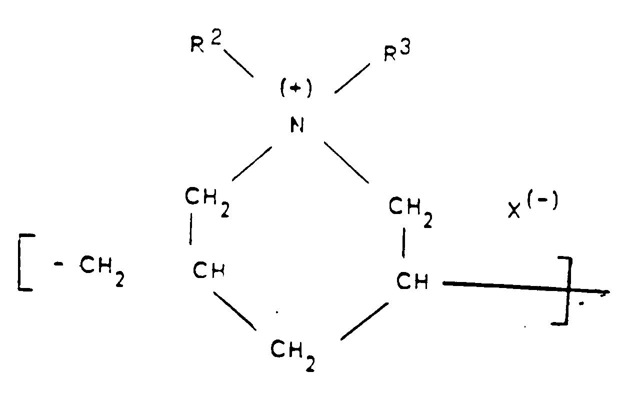 in der R² und R³ Wasserstoff oder Alkylgruppem mit 1 - 4 C-Atomen oder Hydroxyalkylgruppen mit 2 - 4 C-Atomen sind und x(-) ein Chlorid-, Bromid-, Hydrogensulfat-, Methoxy- oder Ethoxysulfat-Anion ist. Die Herstellung solcher Polymeren ist aus US-PS 3.912.808 bekannt. Handelsprodukte dieser Struktur sind z.B. Merquat^{(R)}100 und Merquat^{(R)}550 (Quaternium 41).

Die im Rahmen dieser Erfindung ganz besonders bevorzugten kationischen Polymeren sind Copolymere aus Dimethyldiallylammoniumchlorid und Acrylsäure. Als entsprechendes Handelsprodukt ist Merquat^{(R)}280 (Polyquaternium 22) zu nennen.

Weitere geeignete kationische Polymere sind z.B. Celluloseether, deren Anhydroglucoseeinheiten jeweils 1 - 3 über Ethersauerstoff gebundene Substituenten mit quartären Ammoniumgruppen tragen. Solche Polymeren sind z.B. aus US-PS 3.472.840 bekannt. Ein Handelsprodukt mit dieser Struktur ist z.B. Polymer JR^{(R)}400.

Weitere besonders geeignete kationische Polymeren sind z.B. die aus der US-PS 3.910.862 bekannten und z.B. unter der Handelsbezeichnung Gafquat^{(R)}734 und 755 erhältlichen quartären Polyvinylpyrrolidon-Copolymerisate, die aus US-PS 3.632.559 bekannten und z.B. unter der Handelsbezeichnung Carataretine^{(R)}F 4 erhältlichen Copolymeren aus Adipinsäure und Dimethylaminohydroxypropyl-diethylentriamin und die aus US-PS 4.157.388 bekannten und z.B. unter der Handelsbezeichnung Mirapol^{(R)}A15 erhältlichen quartären polymeren Harnstoffderivate.

Die in den erfindungsgemäßen Haarfärbemitteln einzusetzenden Ethercarbonsäuren entsprechen der Formel I

R¹ - O - (CH₂CH₂O)ₙ - CH₂COOH

, wobei R¹ für eine Alkylgruppe mit 8 - 22 C-Atomen und n für eine Zahl von 1 bis 15, vorzugsweise von 2 bis 5, steht. Die Ethercarbonsäuren können auch in Form ihrer Alkali- oder Ammoniumsalze vorliegen. Solche Produkte sind im Handel z.B. unter der Bezeichnung Akypo RLM 100 oder Akypo RLM 45 N (Fa. Chem-Y) erhältlich.

Fakultativ können weitere - unten näher bezeichnete - Tenside enthalten sein. Alle erfindungsgemäßen Inhaltsstoffe (direktziehender Farbstoff, kationisches Polymer, α-Hydroxysäure, Ethercarbonsäure) können selbstverständlich auch in Form von Gemischen verschiedener Vertreter eines Inhaltsstoffs vorliegen.

Fakultativ zu den direktziehenden Farbstoffen können auch Oxidationsfarbstoffvorprodukte enthalten sein. Oxidationsfarbstoffvorprodukte untergliedern sich in Entwickler- und Kupplerkomponenten. Die Entwicklerkomponenten bilden unter dem Einfluß von Oxidationsmitteln oder Luftsauerstoff untereinander oder unter Kupplung mit einer oder mehreren Kupplerkomponenten die eigentlichen Farbstoffe aus.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Spezielle Vertreter sind beispielsweise p-Toluylendiamin, 2,4,5,6-Tetraaminopyrimidn, p-Aminophenol, N,N-Bis-(2-hydroxyethyl)-p-phenylendiamin` 2-(2,5-Diaminophenyl)-ethanol, 2-(2,5-Diaminophenoxy)-ethanol, 1-Phenyl-3-carboxyamido-4-amino-pyrazolon-5 und 4-Amino-3-methylphenol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, Pyrogallol, 1,5-, 2,7- und 1,7-Dihydroxynaphthalin` 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 2-Chlor-6-methyl-3-aminophenol und 2-Methylresorcin.

Die erfindungsgemäßen wasserhaltigen Haarfärbemittel werden i.a. als kosmetische Zubereitungen, beispielsweise als Cremes, Emulsionen, Gele oder auch tensidhaltige schäumende Lösungen, z.B. Shampoos, Schaumaerosole oder andere Zubereitungen, die für die Anwendung auf dem Haar geeignet sind, konfektioniert.

Übliche Bestandteile solcher wasserhaltiger kosmetischer Zubereitungen sind z. B. Netz- und Emulgiermittel, wie anionische, nichtionische und ampholytische Tenside, z. B. Fettalkoholsulfate, Alkansulfonate, α-Olefinsulfonate, Fettalkoholpolyglykolethersulfate, Ethylenoxidanlagerungsprodukte an Fettalkohole, Fettsäure und Alkylphenole, Sorbitanfettsäureester und Fettsäurepartialglyceride, Fettsäurealkanolamide sowie Verdickungsmittel, wie z. B. Methyl- oder Hydroxyethylcellulose` Stärke, Fettalkohole, Paraffinöle, Fettsäuren, ferner Parfumöle und haarpflegende Zusätze, wie z. B. wasserlösliche kationische ampholytische und anionische Polymere, Proteinderivate, Pantothensäure, Cholesterin, Farbstoffe, Wirkstoffe wie Panthenol, Allantoin, Pyrrolidoncarbonsäuren und deren Salze, Pflanzenextrakte und Vitamine, Lichtschutzmittel, Konsistenzgeber wie Zuckerester, Polyolester oder Polyolalkylether, Wachse, wie Bienenwachs und Montanwachs, Komplexbildner wie EDTA, NTA und Phosphonsäuren, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Perlglanzmittel wie Ethylenglykolmono- und -distearat, Treibmittel wie Propan-Butan-Gemische, N₂O, Dimethylether, CO₂ und Luft sowie Antioxidantien, Reduktionsmittel wie z.B. Thioglykolsäure und deren Derivate, Thiomilchsäure, Cysteamin, Thioäpfelsäure und α-Mercaptoethansulfonsäure, Oxidationsmittel wie Wasserstoffperoxid, Kaliumbromat und Natriumbromat. Die Bestandteile der kosmetischen Träger werden zur Herstellung der erfindungsgemäßen Färbemittel in für diese Zwecke üblichen Mengen eingesetzt, z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die Anwendung der erfindungsgemäßen Haarfärbemittel kann unabhängig von der Art der kosmetischen Zubereitung z. B. als Creme, Gel oder Shampoo im schwach sauren, neutralen oder alkalischen Milieu erfolgen. Bevorzugt ist die Anwendung der Haarfärbemittel in einem pH-Bereich von 6 bis 8. Die Einstellung des pH-Wertes erfolgt mit Hilfe üblicher pH-Stellmittel, z.B. Ammoniak. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Die nachfolgenden Beispiele sollen den Erfindungsgegenstand näher erläutern, ohne ihn jedoch hierauf zu beschränken.

### Beispiele

Es wurden Haarfärbemittel der folgenden Zusammensetzungen hergestellt (Angaben in Gew.-%).

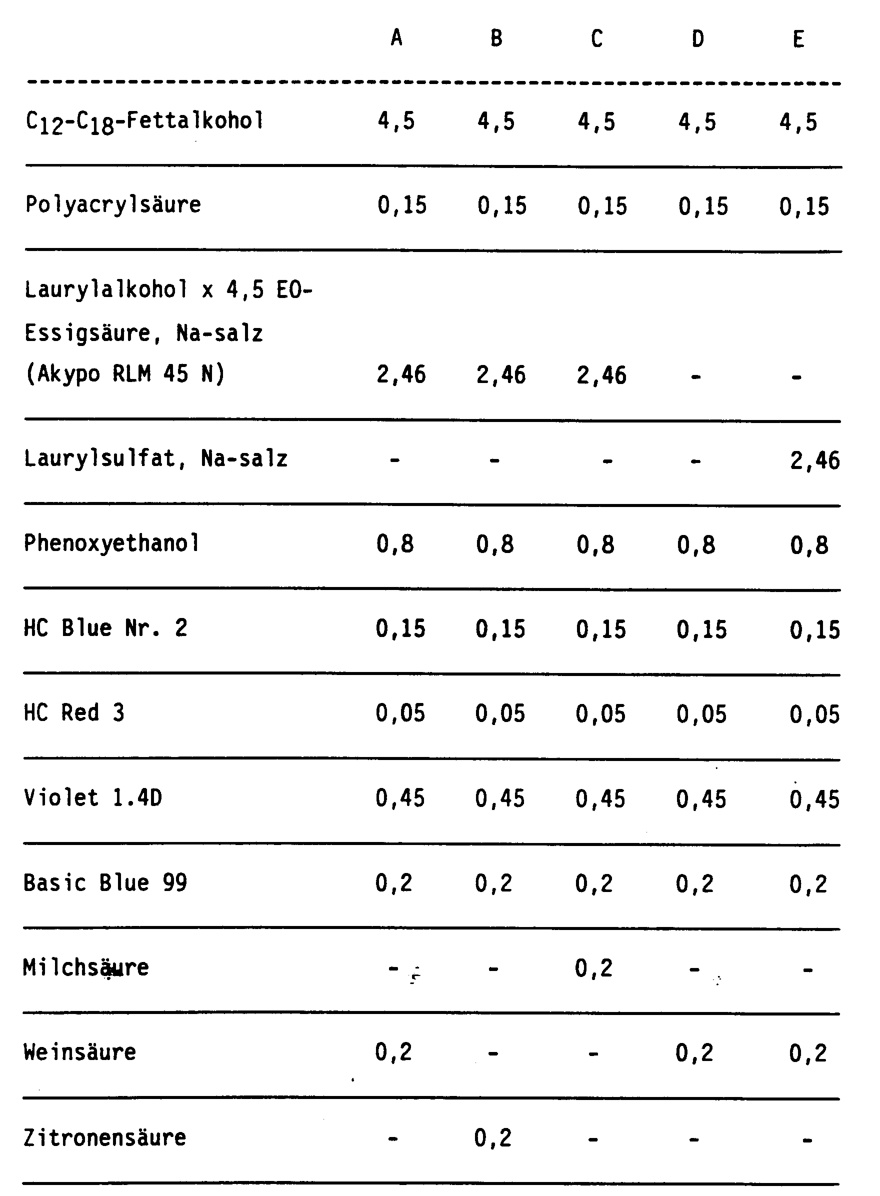

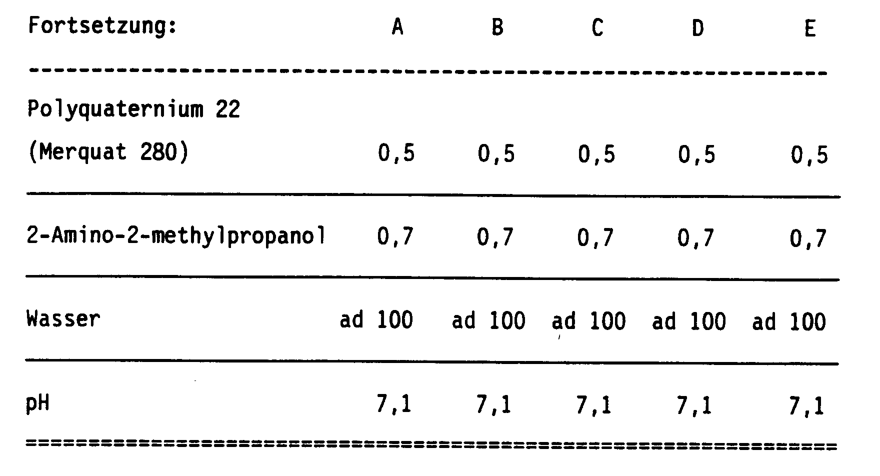

Es wurde eine "Wild-Pflaume" - Nuance erhalten.
Die Zusammensetzungen A - C sind erfindungsgemäß.
D und E dienen zum Vergleich (D enthält keine Ethercarbonsäure; in E ist die Ethercarbonsäure durch Laurylsulfat ersetzt).

### Ermittlung der Farbbrillanz/Leuchtkraft, Farbintensität und des Egalisiervermögens

O.g. Parameter wurden visuell ermittelt:

An einem Modellpanel von je 10 Personen wurden die beiden mit den erfindungsgemäßen Haarfärbemitteln A bis C erzielten Nuancen halbseitig gegen nicht-erfindungsgemäße Rezepturen getestet. 3 weitere Personen beurteilten die Ausfärbungen visuell sowohl bei Tageslicht als auch bei Neonlicht.

Die Bewertungen erfolgten nach einer Skala von 1 (sehr gut) bis 6 (ungenügend).

Die Zusammensetzungen A - E ergeben folgende Ergebnisse:

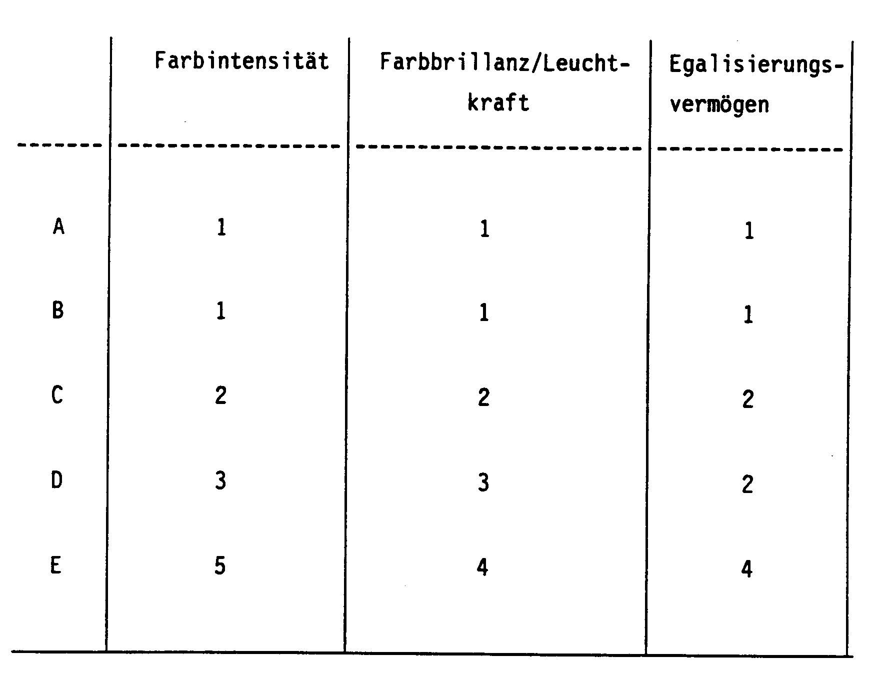

Es zeigt sich, daß die erfindungsgemäßen Zusammensetzungen A - C den nicht erfindungsgemäßen Zusammensetzungen D und E hinsichtlich färbetechnisch relevanter Parameter deutlich überlegen sind.

## Patentansprüche

1. Wasserhaltiges Haarfärbemittel enthaltend
- einen direktziehenden Farbstoff in einer Menge von 0,01 bis 5,0 Gew.-%,
- ein kationisches Polymer in einer Menge von 0,05 bis 2,0 Gew. -%,
- eine α-Hydroxysäure ausgewählt aus der Gruppe Milchsäure, Zitronensäure und Weinsäure bzw. deren Alkali- oder Ammoniumsalzen in einer Menge von 0,05 bis 2,0 Gew.-%,
- eine Ethercarbonsäure der Formel I
R¹ - O - (CH₂CH₂O)ₙ - CH₂COOH (I)
, in der R¹ für eine Alkylgruppe mit 8 bis 22 C-Atomen und n für eine Zahl von 1 bis 15 steht, bzw. deren Alkali- oder Ammoniumsalze in einer Menge von 0,5 bis 8,0 Gew.-%, alle Gew.%-Angaben bezogen auf das gesamte Haarfärbemittel.

2. Haarfärbemittel nach Anspruch 1, dadurch gekennzeichnet, daß die α-Hydroxysäure Weinsäure bzw. dessen Alkali- oder Ammoniumsalz ist.

3. Haarfärbemittel nach Anspruch 1 und 2, dadurch gekennzeichnet, daß es einen pH-Wert von 6 bis 8 aufweist.

4. Haarfärbemittel nach Anspruch 1 bis 3, dadurch gekennzeichnet, daß in der Ethercarbonsäure der Formel I n für eine Zahl von 2 bis 5 steht.

5. Haarfärbemittel nach Anspruch 1 bis 4, dadurch gekennzeichnet, daß das kationische Polymer ein Copolymer aus Dimethyldiallylammoniumchlorid und Acrylsäure ist.

## Claims

1. A water-containing hair colorant containing
- a substantive dye in a quantity of 0.01 to 5.0% by weight,
- a cationic polymer in a quantity of 0.05 to 2.0% by weight,
- an α-hydroxy acid selected from the group consisting of lactic acid, citric acid and tartaric acid or alkali metal or ammonium salts thereof in a quantity of 0.05 to 2.0% by weight,
- an ether carboxylic acid corresponding to formula (I):
R¹ - O - (CH₂CH₂O)ₙ - CH₂COOH (I)
in which R' is an alkyl group containing 8 to 22 carbon atoms and n is a number of 1 to 15,
or alkali metal or ammonium salts thereof in a quantity of 0.5 to 8.0% by weight,
all percentages by weight based on the hair colorant as a whole.

2. A hair colorant as claimed in claim 1, characterized in that the α-hydroxy acid is tartaric acid or an alkali metal or ammonium salt thereof.

3. A hair colorant as claimed in claims 1 and 2, characterized in that it has a pH value of 6 to 8.

4. A hair colorant as claimed in claims 1 to 3, characterized in that, in the ether carboxylic acid of formula (I), n is a number of 2 to 5.

5. A hair colorant as claimed in claims 1 to 4, characterized in that the cationic polymer is a copolymer of dimethyl diallyl ammonium chloride and acrylic acid.

## Revendications

1. Teinture pour cheveux contenant de l'eau, comprenant :
- un colorant à action directe en une quantité allant de 0,01 à 5,0 % en poids,
- un polymère cationique en une quantité allant de 0,05 à 2,0 % en poids,
- un acide α-hydroxylé choisi dans le groupe de l'acide lactique, de l'acide citrique et de l'acide tartrique ou leurs sels de métal alcalin ou d'ammonium, en une quantité allant de 0,05 à 2,0 % en poids,
- un acide éthercarboxylique de formule I
R¹ - O - (CH₂CH₂O)ₙ - CH₂COOH (I)
dans laquelle R¹ représente un radical alkyle ayant de 8 à 22 atomes de carbone et n représente un nombre allant de 1 à 15 ou leurs sels de métal alcalin ou d'ammonium en une quantité de 0,5 à 8,0 % en poids,
toutes les données en % en poids rapportés à l'ensemble de la teinture pour cheveux.

2. Teinture pour cheveux selon la revendication 1,
caractérisée en ce que
l'acide α-hydroxylé est l'acide tartrique ou son sel de métal alcalin ou d'ammonium.

3. Teinture pour cheveux selon les revendications 1 et 2,
caractérisée en ce qu'
elle présente une valeur de pH allant de 6 à 8.

4. Teinture pour cheveux selon les revendications 1 à 3,
caractérisée en ce que
dans l'acide éthercarboxylique de formule I, n représente un nombre allant de 2 à 5.

5. Teinture pour cheveux selon les revendications 1 à 4,
caractérisée en ce que
le polymère cationique est un copolymère à base de chlorure de diméthyldiallylammonium et d'acide acrylique.
